# EUROPEAN PATENT SPECIFICATION

(11) **EP 0 486 522 B1**
(45) Date of publication and mention of the grant of the patent: **07.12.1994**
(21) Application number: 90911384.7
(22) Date of filing: 01.08.1990
(51) Int. Cl.: A61F 13/02

(54) **ADHESIVE DRESSINGS**
HEFTPFLASTER
PANSEMENTS ADHESIFS

(30) Priority: 03.08.1989 GB 8917788
(43) Date of publication of application: 27.05.1992
(73) Proprietor: SMITH & NEPHEW P.L.C., London WC2R 3BP (GB)
(72) Inventor: RAWLINGS, David, Alan, Saffron Walden Essex CB11 4TA (GB); BLOTT, Patrick, Lewis, Hertfordshire CM23 4ER (GB)
(74) Representative: Gilholm, Stephen Philip
(86) International application number: GB9001193
(87) International publication number: WO9101706

(56) References cited:
- EP-A- 0 002 509
- EP-A- 0 099 748
- EP-A- 0 106 440
- EP-A- 0 174 803
- EP-A- 0 236 104
- WO-A-89/06950
- US-A- 4 212 296

## Description

This invention relates to adhesive dressings and particularly to adhesive bandages as would be suitable for first-aid dressings.

A prime requirement for first-aid dressings is that they should be highly conformable and pliable since they have to be used for the dressing of highly rounded surfaces such as fingers as well as for flexing surfaces such as knuckles.

Conventionally first-aid dressings are formed as a relatively bulky but small pad of an absorbent material such as gauze layers adhered to a larger backing sheet made of a woven fabric or filmic material. Usually the pad is covered by layers of coverstock intermediate the pad material on the wound to reduce adherence between the wound eschar and the fibres of the absorbent material.

The manufacturing of such dressings requires need to maintain accurate registry between the coverstock and the pad and the pad and backing layer during the production stage.

Known dressings may also suffer from the disadvantage, that because they are manufactured from highly porous materials, air-borne bacteria can enter the dressing and infect the wound. This problem may be further accentuated where the dressings are not water proof and water-borne bacteria and viruses can enter or leave the dressings.

US-A-4,212,296 discloses a conformable wound dressing comprising an absorbent layer with a planar wound facing surface, an absorbent pad comprising a polymeric foam and a layer of a moisture vapour permeable material over the opposed surface of the absorbent layer wherein the thickness of the absorbent layer at two opposed margins is substantially less than the thickness of the absorbent layer between said margins.

However, the wound facing surface of the absorbent layer comprises a continuous adhesive layer.

We have now found that the problems associated with manufacture and bacterial contamination may be reduced by highly pliable and conformable dressing comprising a composite of coextensive layers.

According to the present invention there is provided a conformable wound dressing comprising an absorbent layer with a planar wound facing surface comprising polymeric foam, characterised in that the dressing comprises a wound facing discontinuous adhesive layer over said surface of the absorbent layer and a layer of a liquid impervious moisture vapour permeable material over the opposed surface of the absorbent layer wherein the thickness of the absorbent layer at two opposed margins is substantially less than the thickness of the absorbent layer between said margins.

The dressings of the invention may be in the form of a composite comprising three coextensive layers, a top moisture vapour permeable layer, an intermediate absorbent layer and a bottom or wound facing adhesive layer.

In an alternative embodiment a fourth or further intermediate layer, which is coextensive with at least the absorbent layer, may be positioned between the absorbent and adhesive layers. This fourth layer should be a discontinuous layer so as not to impair the performance of the absorbent layer.

A feature of the dressings of the invention is that opposed margins are substantially thinner than the region between the dressing. This feature not only enhances the conformability of the dressing since the flexural rigidity of the dressing is reduced at the margins but also minimises the risk of the dressing being caught at the edge and inadvertantly lifted.

The term 'opposed margins' not only encompasses those embodiments where there are margins on separate, clearly defined edges forming the perimeter of the dressing but also those embodiments where there is a single continuous margin around the perimeter of the dressing and no discernable edges eg. the perimeter of a circular or oval dressing. In the latter case 'opposed margins' refers to the margin at opposed points or positions on the dressing. Thus, for circular dressings the opposed margins are the margins at diametrically opposed positions.

The thickness of the opposed margins of the absorbent layer should preferably be no greater than two-thirds of the thickness of the layer between the margins. More preferably the thickness of margins should be less than half the thickness of the layer between the margins. The thickness of the dressing may be reduced step-wise or continuously towards the edge margins. For example the material comprising the absorbent layer may have chamfered edges.

For dressings, suitable for use as first-aid bandages, the thickness of the foam-containing layer in the region between the edges may be upto about 5mm thick, preferably upto about 2.5mm thick. For specialist applications or for larger dressings such as ward dressings, the thickness of the region between the edges may be greater than 5mm for example upto 2.5cm. The thickness of the edges may, for the dressing of the invention be no greater than about 10% of the thickness of the intermediate region.

Normally, the layers of the dressing are attached in a contiguous manner so as to form a laminate.

Wound dressings of the invention are preferably moisture vapour permeable and can suitably have an moisture vapour transmission rate of at least 300, aptly from 300 to 5000 grams and preferably 500 to 2000 grams/square meter/24 hours at 37.5°C at 100% to 10% relative humidity difference. It has been found that such moisture vapour transmission rates will allow the wound under the dressing to heal under moist conditions without causing the skin surrounding the wound to macerate.

The moisture vapour transmission rates (MVTR) as used herein were defined as follows:
Discs of the material under test are clamped over Payne Permeability Cups (flanged metal cups) using sealing rings and screw clamps. The exposed surface area of the test sample is 10cm². Each cup contains approximately 10ml of distilled water.

After weighing the cups are placed in a fan assisted electric oven which is maintained at 37±1°C. The relative humidity within the oven is maintained at approximately 10% by placing 1Kg of anhydrous 3-8 mesh calcium chloride on the floor of the oven.

The cups are removed after a predetermined period of time allowed to cool for 20 minutes and re-weighed. The MVTR of the test material is calculated from the weight loss and expressed in units of grams of weight per square metre per 24 hours.

The adhesives employed in the present invention are suitably those which do not adhere to the moist surface of the healing wound. The adhesive on the wound facing surface of dressings of the invention then allows these dressings to be adhered to the skin around the wound site.

The adhesive may be applied as a coating on the absorbent layer or on a intermediate discontinuous layer. In another embodiment the adhesive may be formed in situ on the intermediate or absorbent surfaces.

The adhesive may be a discontinuous layer. Such a layer may be randomly discontinuous, such as randomly distributed spots or a layer of a porous adhesive. Alternatively, the discontinuous adhesive layer may be a regular patterned layer in the form of regularly arranged spots or lines or in a grid arrangement.

Where the adhesive is coated on to the discontinuous layer, the adhesive itself may be a continuous coating on the surfaces of the intermediate layer. The adhesive layer will itself be discontinuous, in which the discontinuities are in registry with the discontinuities in the intermediate layer.

In an alternative embodiment of the invention, the adhesive layer need not be coextensive with the absorbent layer. It is desirable that adhesive be present at the edges of the dressing. However the adhesive layer need not be over the whole wound facing surface of the absorbent or intermediate layer. In this embodiment the adhesive layer may comprise a continuous or discontinuous adhesive coating or layer. The adhesive may be coloured differently from that of the absorbent layer or intermediate layer to provide a visual distinction. The absorbent layer or intermediate layer may therefor be the direct wound-contacting layer; with a peripheral layer of adhesive for securing the dressing to intact tissue.

It is preferred that at least 10% of the wound facing surface of the absorbent or intermediate layer be covered with adhesive and that the adhesive be present at all the peripheral regions of the wound facing layer. However, it may be desired that the free area is as little as 40%.

Suitable adhesive layers for the wound facing layer of dressings of the invention have a thickness of 15 microns to 75 microns and preferably a thickness of 25 to 50 microns. Suitable adhesives for the wound facing layer can be any of those pressure sensitive adhesives normally used for adhesive surgical or medical dressings. Preferred pressure sensitive adhesives comprise acrylate ester copolymers and polyvinyl ethyl ether adhesives, such as those disclosed in United Kingdom Patent Specification No. 2070631 and European Patent Specification Nos. 0099675 and 0194881.

The material employed for the discontinuous layer intermediate the adhesive and the foam absorbent layer may be a conformable, elastomeric film provided with apertures or may be a plurality of conformable, elastomeric polymer strands laid down to a net of desired shape, size and configuration. In another embodiment the discontinuous layer may be formed from a non-woven material.

Aptly the discontinuous layer is formed from a polymeric film. The film may be perforated, apertured or cut to provided. Alternatively the film may be subjected to physical treatments to form a net.

The conformable elastomeric discontinuous film or net allows wound exudate to pass to the absorbent foam layer but prevents the absorbent layer making direct contact with the wound surface.

Preferably, the discontinuous layer used in this invention is an integral net, that is a net with strands and junctions which have been formed integrally from a unitary film during manufacture.

Preferably the discontinuous layer is sufficiently conformable to allow the wound dressing to conform with the body contours and thereby maintain overall contact with the wound surface to ensure that exudate from the wound is absorbed.

It is also desirable that the discontinuous layer should be sufficiently elastically extensible to adjust to any dimensional changes in the absorbent layer which may occur, for example, by expansion on liquid uptake.

Suitable discontinuous layers will have elongation at break of 100% to 800% desirably 200% to 750% and preferably 300% to 700% when measured as a 2.5cm wide strip at a 30cm/minute strain rate at 20°C.

Normally the discontinuous layer of elastomer is made of a pharmaceutically acceptable water insoluble elastomer.

Suitable elastomers for use in manufacturing the discontinuous layer include polyurethanes, polybutadiene and the like.

Polymer blends having a continuous phase of an elastomer and a discontinuous phase of a polymer which is incompatible with the elastomer may also be used for the intermediate layer eg. net. Suitable blends include those of elastomers such as polyurethane or ethylene-vinyl acetate copolymer with incompatible polymers such as olefine, eg. polystyrene.

The preferred material for the nets are thermoplastic polyurethanes.

Preferred thermoplastic polyurethanes are linear polyurethanes containing polyether or polyester groups. Suitable linear polyester polyurethanes are disclosed in United States Patent Specification No. 2871218. Suitable linear polyether polyurethanes are disclosed in United States Patent Specification No. 2899411. Favoured thermoplastic polyurethanes include Estanes from B.F. Goodrich Corp. Preferred solution casting grades are Estane 5714F1, 5702 and 5703. A preferred extrusion grade is Estane 580201.

Suitable polybutadienes are 1,2 polybutadienes. Favoured 1,2 polybutadienes contain a major amount of syndiotactic 1,2 polybutadiene, have a crystallinity of 25% to 30% and an average molecular weight in excess of 100,000. Preferred 1,2 polybutadienes are known as RB 810, RB820 and RB 830 made by Japan Synthetic Rubber Company.

The net of the discontinuous layer of the dressing can have any convenient form depending on the chosen arrangement of strand, junctions and aperture areas and also their shapes and relative size.

The number and size of the apertures in the net will be sufficient to allow the wound exudate to pass through the film to the absorbent layer. Most aptly the net is adapted so that the size of apertures in combination with the thickness of the film prevent the absorbent layer contacting the wound surface. Suitable nets have apertures with a dimension of upto 4mm. Generally the apertured dimension will exceed 0.5mm, for example from 0.05 to 4mm, more aptly from 0.05 to 2.5mm or 0.05 to 2.0, and preferably from 0.1 to 2.5mm. Suitable nets have a thickness of at least 0.04mm. Aptly the thickness will be upto 2.5mm, eg. from 0.01 to 2.5mm, typically of 0.01 to 0.25mm and preferably of 0.05 to 0.5mm. Aptly the nets of the invention have at least 4 and more aptly upto 40 apertures per cm. Favoured nets of the invention have 4 to 40 apertures per cm with dimension of 0.05 to 2.5mm. The wound face of the net may have upto 80% of its area void. The wound face of the net will suitably have 15 to 80% of its area void (the apertures). More suitably 25 to 75% of its area will be void and most suitably will have 35 to 65% of its area void.

The net of the wound dressing of the invention can have any convenient form depending on the chosen arrangement of strand, juncture and hole areas and also their shapes and relative size.

In one preferred form the net consists essentially of longitudinal and transverse strands intersecting at right angles to give a square grid hole pattern.

Suitable nets of this type aptly have 2 to 50 strands per cm, desirably 4 to 40 strands per cm and preferably 2 to 24 strands per cm in both longitudinal and transverse directions.

Variations on the square grid pattern can give other desirable forms of the integral net. Unequal density of strands in either the longitudinal or transverse directions will give rectangular hole areas. Continuous parallel strands in one direction with a staggered arrangement of connecting strands in the other direction will give a "brick-work" pattern. Other apt forms of the integral polymer nets can have strands at an angle to the longitudinal or transverse direction (that is diagonal strands). Another preferred form of the integral polymer net can have a staggered arrangment of circular or approximately circular (for example hexagonal) arrangements of strands and hole areas. The integral polymer net can be in the form of a mixed pattern of two or more of the arrangements as desired.

The apertured film or net used in this invention aptly will have a weight of 10gsm to 80gsm and preferably will have a weight of 15gsm to 50gsm.

The adhesive layer or combination of adhesive and discontinuous layer is preferably distensible such that distortion of the dressing does not occur during dimensional changes in the absorbent layer which may occur, for example by expansion in liquid uptake or on body surface movement eg. flexing or stretching over a knuckle or elbow.

The absorbent employed in the absorbent layer of the dressings of the present invention is a polymer foam. The foam is preferably a highly conformable hydrophilic foam, more preferably an open celled foam.

The conformable hydrophilic polymer open cell absorbent layer used in dressings of the invention is capable of absorbing wound exudate. It is desirable that the hydrophilic polymer form layer absorbs the wound exudate rapidly as this enhances the low adherency properties of the absorbent pad. Such rapid absorption prevents undesirable pooling of exudate between the dressing and the wound.

The ability of open cell hydrophilic polymer foam layers to absorb and retain fluids depends to some extent on the size of the foam cells, the porosity of the foam and the thickness of the foam layer.

Suitable open cell hydrophilic foams of dressings of the invention have a cell size of 30 microns to 700 microns and preferably a cell size of 50 microns to 500 microns. Apt open cell hydrophilic foams of dressings of the invention have 20% to 70% and preferably 30% to 60% of the total membrane area of the cells as membrane openings. Such open cell foams permit transport of fluid and cellular debris into and within the foam.

Apt foams may be polyurethane, carboxylated butadiene styrene rubber, polyacrylate or the like foam. Such foams may be made of hydrophilic materials per se or may be treated to render them hydrophilic, for example with surfactants. It is much preferred to use foams which are made of polymer which is itself hydrophilic as it has been found the exudate is less likely to coagulate rapidly.

The use of such foams of hydrophilic polymer in the absorbent pad of dressings of the invention can allow the wound to be maintained in a moist condition even when the exudate produced has been absorbed and removed from the wound surface.

Favoured hydrophilic polymer foams are hydrophilic polyurethane and especially those which are made of crosslinked hydrophilic polyurethane. Preferred foams can be made by reacting a hydrophilic isocyanate terminated polyether prepolymer with water.

Suitable hydrophilic polyurethane foams of this type include those known as Hypol foams. Hypol foams can be made from Hypol hydrophilic prepolymers marketed by W.R. Grace and Co.

The conformable hydrophilic polyurethane foam can be made by mixing together an isocyanate terminated polyether having functionality of more than two with a surfactant and water and casting the mixture onto a surface. This surface advantageously may be the intermediate discontinuous layer.

Preferred isocyanate terminated polyethers include Hypol FHP 2000, 2001, 3000, 3001, 2002 and 2000HD marketed by W.R. Grace and Co. Hypols are described in a booklet published by W.R. Grace and Co. "Hypol: foamable hydrophilic polymers - laboratory procedures and foam formulations". Their preparation and use are disclosed in British Patent Specifications Nos. 1429711 and 1507232.

Suitable surfactants for forming conformable hydrophilic polymer foams include non-ionic surfactants. Favoured non-ionic surfactants are oxypropylene-oxyethylene block copolymers known as Pluronic marketed by BASF Wyandotte. Preferred Pluronics include L65, F87, P38, P75 and L62. Another favoured non-ionic surfactant is a polyoxyethylene stearyl ether known as Brij 72 marketed by Honywell Atlas.

To prepare a suitable foam 100 parts by weight of Hypol FHP 2000, 2001, 3000, 3001, 2002 or 2000HD is mixed with 0.3 to 7 parts by weight of surfactant or mixtures of surfactants and 30 to 300 parts by weight of water and the foaming mixture cast onto a surface. Typical foaming mixtures have a cream time of about 20 s, a rise time of about 250 s and a cure time of about 400 s.

A preferred foam for use in the absorbent layer of the dressings of the invention is disclosed in our United Kingdom Patent Specification No. 2188055 which inter alia there is described a hydrophilic polyurethane foam comprising residues derived from a polyalkylene glycol mono alkyl or mono alkaryl ether. Such foams can be formed by reacting with water the reaction product of polyisocyanate which has a functionality of greater than 2 and polyalkylene glycol mono alkyl or alkaryl ether.

Preferred polyalkylene glycol mono alkaryl ethers are those in which the alkylene group contains upto 4 carbon atoms. More preferably the alkylene group is ethylene.

Suitable polyalkylene glycol mono alkyl ethers for forming the reaction product are those in which the alkyl group contains 1 to 20 carbon atoms. Alkylene favoured ethers are those in which the alkyl group is a methyl group. Another class of preferred polyalkylene glycol mono alkyl ethers are those in which the alkyl group contains 10 to 18 carbon atoms, eg. lauryl or cetyl.

Suitable polyalkylene glycol mono alkaryl ethers include those in which the aryl moeity is phenyl. Preferred ethers are those in which the alkyl moeity contains from 1 to 20 carbon atoms eg. octyl or nonyl.

The polyalkylene glycol mono alkyl or alkaryl ether can suitably have an average molecular weight of 180 to 6000. Suitable ethers for forming reaction products used to prepare flexible foams of the invention have an average molecular weight of 180 to 1300 and preferably have an average molecular weight of 350 to 1000.

Suitable ethers for forming reaction products used to prepare stiff foams of the invention have an average molecular weight of 1500 to 6000 and preferably have an average weight of 3000 to 5000.

Apt ethers are polyethylene glycol mono lauryl ethers having an average molecular weight of approximately 1090 and 360 known as Brij 35 and Brij 30 respectively available from Honeywell Atlas and polyethylene glycol mono methyl ethers having an average molecular weight of approximately 500 and 5000 known as PEG monomethylether molecular weight 550 and 5000 respectively available from Aldrich Chemicals.

Suitable polyethylene glycol mono nonyl phenyl ethers are commercially available under the Trade names Antarox CO-320 and Antarox CO-990. Apt polyethylene glycol mono nonyl phenyl ethers, having an average molecular weight of approximately 440 and known as Antarox CO-520 and Co-990 respectively available from GAF (Great Britain) Co. Limited.

The polyethylene glycol mono alkyl or alkaryl ether used in the invention will normally contain water. It is preferred however, that the ether contains less than 1% by weight of water to limit the number of urea groups formed in the reaction with the polyisocyanate.

The polyisocyanate used for forming the reaction product will have a functionality greater than 2 for example 2 to 5 and will preferably have a functionality of 2.2 to 3.5. Suitable polyisocyanates include aliphatic and aromatic polyisocyanates. Preferred polyisocyanates are aliphatic polyisocyanate. Aliphatic polyisocyanates are usually liquid at ambient room temperature and therefore are convenient to use in a liquid reaction mixture. An apt aliphatic polyisocyanate for use in the invention is a biuret of 1,6 hexamethylene diisocyanate which has a functionality of 2.6 known as Desmodur N100 available from Bayer A.G.

Favoured aromatic polyisocyanates for forming the reaction product are polymeric methylene diisocyanates. Polymeric methylene diisocyanates comprise a mixture of 4,4'-diphenyl methane diisocyanates and one or more of polymeric homologues. Apt polymeric methylene diisocyanates are known as suprasec VM 20, VM 50, DND and VM 90 available from ICI and have a functionality of 2.13, 2.49, 2.70 and 2.90 respectively.

The reaction product suitable for use in the invention can be a reaction product of one or more polyisocyanates and one or more polyalkylene glycol mono alkyl are aryl alkyl ethers, including mixed alkyl and alkaryl ethers. The reaction product may advantageously be formed using a chain extender.

Suitable chain extenders for use in forming the reaction product include ethane diol, 1.3 propane diol and 1.4 butane diol.

The conformable moisture vapour transmitting outer layer of dressings of the invention when present can be continuous or discontinuous.

A preferred moisture vapour transmitting outer layer is a continuous conformable film. The continuous moisture vapour transmitting conformable film outer layer of the wound dressing of the invention may be used to regulate the moisture loss from the wound area under the dressing and also to act as a barrier to bacteria so that bacteria on the outside surface of the dressing cannot penetrate to the wound area.

Suitable continuous conformable films will have a moisture vapour transmission rate of at least 300, aptly from 300 to 5000 grams preferably 500 to 2000 grams/square meter/24 hrs at 37.5°C at 100% to 10% relative humidity difference. It has been found that such moisture vapour transmission rate of the continuous film allow the wound under the dressing to heal under moist conditions without causing the skin surrounding the wound to macerate.

Suitable moisture vapour transmitting continuous films can be made of polyurethane or copolymers of alkoxy alkyl acrylates or methacrylates such as those disclosed in British Patent No. 1280631. Apt polyurethanes and polyurethane films, particularly highly moisture vapour permeable polymers and foams are also disclosed in European Patent Specification No. 0091800.

The continuous moisture vapour transmitting film can be a conformable polyurethane incompatible polymer blend film containing voids. Suitable conformable polyurethane blend films are disclosed in United Kingdom Patent Application GB 2081721A. A preferred film is a polymer blend of polyurethane and high impact polystyrene.

An apt conformable moisture vapour transmitting outer layer comprises a microporous film. The conformable film microporous outer layer of the wound dressing of the invention may be used to regulate the moisture loss from the wound area under the dressing and also to act as a barrier to bacteria to delay or prevent bacteria on the outside surface of the dresssing penetrating to the wound area.

Suitable conformable microporous films will have a moisture vapour transmission rate of 300 to 5000 grams preferably 500 to 4000 grams/square meter/24 hrs at 37.5°C at 100% to 10% relative humidity difference.

Suitable conformable microporous films have pore diameter of less than 2 microns desirably less than 0.6 microns and preferably less 0.1 microns. Such microporous films should have pore diameter of greater than 0.01 microns.

Suitable conformable microporous films have a thickness of 25 to 400 microns preferably 50 to 300 microns. The conformable microporous film will be made of a polymer.

Suitable polymers include polether-polyamide copolymers such as those marketed under the name PEBAX (CATCOCHEM SA) containing a particulate filler, eg chalk, plasticised polyvinyl chloride, polyurethane elastomers and ethylene vinyl acetate copolymer elastomers.

A favoured conformable microporous film comprises a microporous plasticised polyvinyl chloride film having an average pore diameter of less than 2 microns, a thickness of 250 to 300 microns and a moisture vapour transmission rate of 3000 to 5000 g/m²/24 hours at 37.5°C at a relative humidity difference of 100% to 10% relative humidity.

The conformable moisture transmitting outer layer of wound dressings of the invention may also comprise a moisture vapour transmitting adhesive layer to bond the outer layer to the layer of open cell foam. The adhesive layers can be continuous or discontinuous.

Suitable adhesives which are moisture vapour transmitting as a continuous layer include various acrylate ester copolymer and polyvinyl ether pressure sensitive adhesives for example as disclosed in British Patent No. 1280631. Favoured pressure sensitive adhesives comprise copolymers of an acrylate ester with acrylic acid for example as disclosed in United Kingdom Application GB 2070631.

The wound dressing of the invention can contain a topically effective medicament. Most suitably the medicament is an antibacterial agent. Preferably the antibacterial agent is a broad spectrum antibacterial agent such as silver salt for example silver sulphadiazine, an acceptable iodine source such as povidone iodine (also called polyvinyl pyrrolidone iodine or PVP/I), chlorhexidine salts such as the gluconate, acetate, hydrochloride or the like salts or quaternary antibacterial agents such as benzalkonium chloride or the like.

The medicament can be located in the foam layer or in the adhesive coating.

The medicament is preferably located in the foam layer of the dressing.

Preferred amounts of suitable medicaments for incorporation into the foam layer of the dressing of the invention are disclosed in the aforementioned patent applications.

The foam in the absorbent layer may also contain a supersorber. Suitable supersorbers are well known and can include starch and other cellulosic materials such as cross-linked methyl cellulose, as well as known materials containing acrylic unsaturation.

The wound dressing of this invention may be in any convenient form of shape or size. In a preferred form the wound dressing is a pad of rectangular, oval or circular shape. In another preferred form the wound dressing can be an elongate strip which may be used as a bandage or may be used to prepare smaller dressings. The dressings may also be of irregular shape for use on flexing or bending surfaces such as knuckles, knees and elbows.

The thinner margins of the dressing may comprise as little as 10% of the total dressing surface area. However, the margins may comprise upto, for example 90% of the total dressing body contacting surface area and may typically comprise from 20 to 60% of dressing body contacting surface.

In one arrangement, the adhesive layer may be arranged to cover only the thinner marginal regions of the dressing, a centrally disposed adhesive free area of the body contacting surface being in registry with the thickened area of the absorbent layer and defining an adhesive free wound contact area.

The width of the margin need not be the same around the whole periphery of the dressing. For example, a generally rectangular or oblate dressing may have opposed margins on one axis which are wider than the margins on the minor axis. As an illustration, a rectangular dressing having body contacting dimensions of 10cm x 2cm may have a centrally disposed thickened region measuring 6cm x 1cm, the margins of the major axis being 3cm wide whilst those on the minor axis being only 0.5cm wide. In this way, the conformability of the dressing is not compromised since the thickened area of the absorbent layer is restricted to the cushioned wound contacting part of the dressing and the thinner edges which bear the majority of the adhesive layer can function to provide the anchorage for the dressing. Dressings of this construction have advantages where, for example, the dressing is applied to a finger, and the ends of the dressing overlap each other. The total thickness of the overlapped regions of the dressing is therefore reduced to a minimum.

Although the dressings of the invention are suitable as first-aid dressings or bandages, they also have use as medical or wound dressings.

The dressings of the present invention have a primary use in the field of first-aid and may be employed in the home and workplace for the primary dressing of wounds and contusions which may cause minor bleeding such as cuts and abrasions, which do not produce large amounts of wound exudate.

The dressings may be supplied in a variety of shapes and sizes for the dressing of lesions ranging from small cuts, for example on the finger to large skin grazes on, for example, the elbow or knee. The dressings may be square, rectangular, round, oval or oblate in shape. The dressings may be of a specialised shape, for example having a number of fingers extending from a main or central wound contacting region for the dressing of wound on the knuckle. The central part of the dressing is placed over the wound on the knuckle and the fingers, which have adhesive on the body facing surface and the finger portion adhered to the adjacent fingers and back of the hand.

Dressings of this configuration allow the hand to flex naturally and freely without the risk of the dressing becoming detached.

The overall dimension of dressings or adhesive bandages in accordance with the invention may be as small as 1cm x 1cm upto 10cm x 10cm. Alternatively the dressing may be supplied in roll form, with the adhesive preferably disposed along the major edges of the roll and the central area between the adhesive coated margins free of adhesive.

The dressings of the present invention may also be employed as wound dressings for the dressing of wounds and lesions which do not produce large amounts of exudate. Such dressings have application as post-operative dressings for the covering of closed surgical incisions and for wounds treated in hospital casualty units, which require protection but are bleeding profusely or producing large amounts of exudate.

Such wound dressings tend to be of a larger size than those dressings used for first-aid application in the home and workplace. Wound dressings may be required in sizes ranging from that of the larger first-aid dressing upto for example 50cm x 20cm.

It is desirable that the wound dressing of this invention is sterile. The wound dressing of the invention is advantageously provided in bacteria impervious pouches. Such packed forms can be prepared under aseptic conditions or alternatively sterilised after packing by a conventional procedure. A favoured sterilisation procedure is heat sterilisation, for example by steam. Other favoured procedures are ethylene oxide sterilisation or gamma irradiation.

In another aspect the present invention provides a process of making a wound dressing of the invention which comprises bringing together a layer of a liquid impervious moisture vapour permeable layer, an absorbent layer comprising polymeric foam and wound facing layer with adhesive on its wound facing surface.

The absorbent layer may be produced by foaming a suitable polymer into a mould to produce the desired shape, by casting into a block and cutting the desired shape before combination with the other components or by casting alone or with the other components and thereafter shaping the composite, for example by embossing, to form an absorbent layer having thin peripheral edges and a thicker region between the edges.

Normally the bringing together of the layers will be a lamination process. Such lamination processes can also be used to form wound dressings with a conformable moisture vapour transmitting outer layer.

The adhesive can be coated onto the wound facing surface of the discontinuous layer before, during or after the layer has been laminated to the foam layer or directly onto the wound facing surface of the foam. In a preferred process the adhesive in a flowable state is cast into the recesses of a release coated surface having a pattern of discrete raised areas and interconnected recessed areas and the net layer formed in a similar manner on the adhesive layer.

Preferred casting surfaces are embossed polymer sheets. Suitable embossed polymer sheets are disclosed in the aforementioned patent applications.

The adhesive surface of wound dresssings of the invention will usually be provided with a release coated protector. The release coated protector can be the embossed sheet carrier used for forming the adhesive coated net layer. Other suitable release coated protectors include silicone coated release papers such as Steralease paper Nos. 15 and 67 made by Sterling Coated Papers Limited.

The dressings of the invention may be readily manufactured by continuous production techniques. Thus a moisture vapour permeable film and a polymer net may be run in together through the nip of two rollers or a single roller and a flat bed and a polyurethane foam injected into the nip between the film and net. The foam may be formed in situ at the point of injection by mixing a suitable isocyanate prepolymer as hereinbefore described with, for example, water. The three-layer composite may then be passed through an embossing station where the composite is embossed, prior to complete curing of the foam, to produce thinner regions in the foam layer. After curing of the foam is completed, the embossed composite may then be transfer coated with a suitable adhesive, pre-coated on a releasable carrier sheet. Once the adhesive has been transferred and the carrier sheet removed conventional protector papers for the adhesive may be run onto the adhesive surface, according to conventional techniques. Finally the dressing may be stamped out by cutting through the composite at the thinner embossed regions, and the individual dressings packaged.

If desired the dressings may be sterilized during or at the completion of the manufacturing and packaging process.

The invention will be further described and illustrated by the accompanying drawings in which:

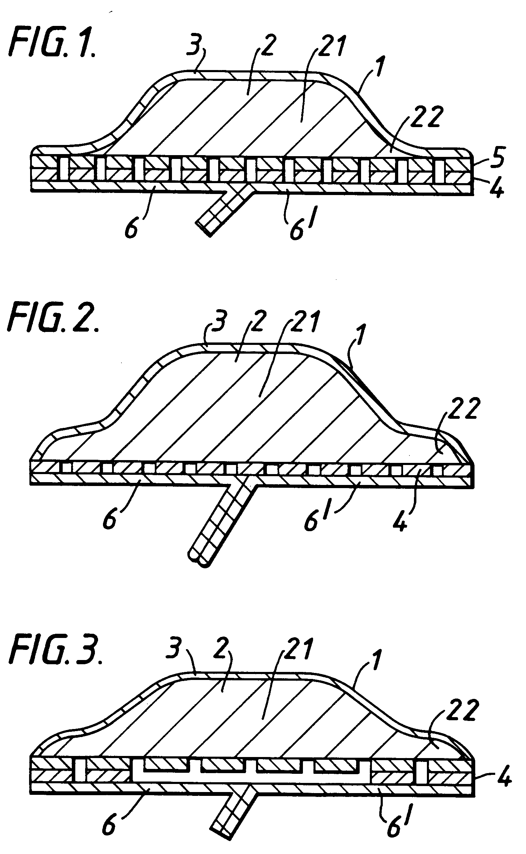

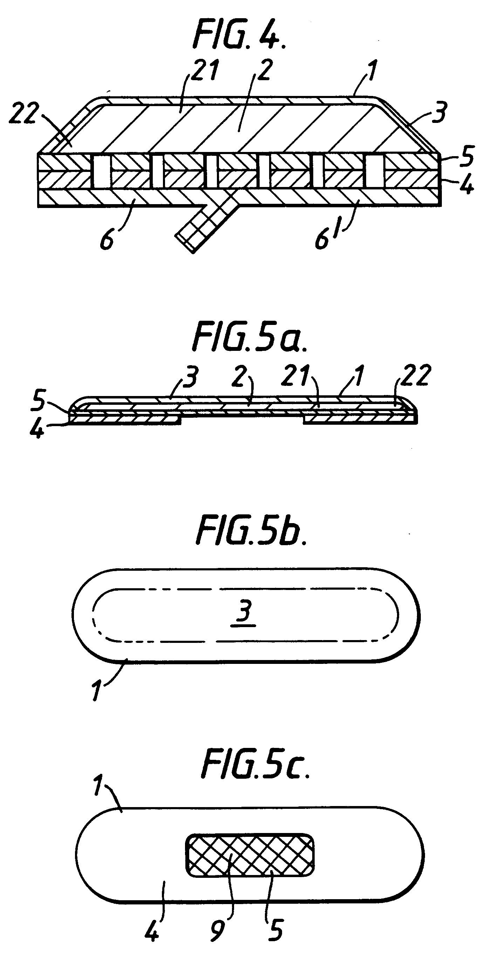

Figures 1 to 4 are schematic cross-sectional views of dressing of the present invention and Figures 5a, 5b and 5c are respectively a cross-sectional view, top view and bottom view of a dressing in accordance with the invention.

Referring to the drawings, a polymeric foam absorbent layer 2 of a dressing 1 is provided with a thickened central region 21 and thinner edge or marginal regions 22. On the top surface of the foam layer 2 is a sheet of liquid impervious water vapour permeable material 3.

In the embodiment shown in Figure 2 a discontinuous layer of adhesive 4 is coated directly onto the absorbent foam 2. However, the adhesive may be formed as a discontinuous layer by coating an intermediate discontinuous layer 5 (Figures 1, 3, 4 and 5a) with adhesive 4 which is then laminated or bonded to the absorbent layer 2. The adhesive may be generally over the whole of the undersurface of the dressing (Figures 1, 2 and 4) or the dressing may be provided centrally with an adhesive-free area 9.

The body contacting surface of the adhesive layer 5 is covered with a pair of release protectors made, for example, from silane coated paper.

The invention is now further illustrated by the following Examples:

### Example 1

A composite was produced by laminating a polyester-polyamide copolymer (Pebax) film, a hydrophilic polyurethane foam, and a polyurethane net through a nip defined by a rotating roller and a flat bed. The nip gap was 100µm and the roller and bed were maintained at ambient temperature.

The polyester-polyamide film (sold under the Trade Name Pebax 2533 Grade SA00) was filled (50% by weight) with chalk and stretched at a draw ratio of 5:1 in both machine and transverse directions to produce a microvoided product having an upright MVTR of about 3000 and thickness between 0.04 and 0.06mm.

The polyurethane net was prepared according to the method described in the Examples of European Patent No. 0059048 and then crushed to a thickness of 15µm.

The polyurethane foam was prepared by first producing a prepolymer according to Example 1 of United Kingdom Patent Specification No. 2188055 and then foaming the prepolymer at the nip of the roller and the bed in accordance with the procedure of Example 7 of United Kingdom 2188055.

After passing through the nip the foam was allowed to gel, and the composite embossed with an oblate punch, heated to 100°C to define a dressing 6cm x 2.5cm with a periphery 1mm thick and a central region 2.5mm thick. The thinner peripheral region extended inwards for about 3.0mm.

After the polyurethane had cured, a pattern spread adhesive, prepared as described in Example 4 of European Patent Specification No. 0194881, was transfer coated onto the polyurethane net side of the composite to give an adhesive coated product. The adhesive coating weight was 43g m⁻² to give an adhesive layer of 27g m⁻².

The adhesive surface was then covered with release papers and the product finally cut out of the composite sheet, through the embossed region, to produce a first-aid dressing.

### Example 2

The procedure of Example 1 was repeated except that the Estane net was omitted and the adhesive was coated directly onto the polyurethane foam by pattern spreading the adhesive. The coating weight of adhesive on the net was 40 grams/m². The coating weight of the adhesive over the dressing surface was 27g/m².

### Example 3

The procedure of Example 1 was repeated except that the moisture vapour permeable film was prepared from a blend of polyurethane and high impact polystyrene prepared in accordance with GB-A-2081721.

### Example 4

The procedure of Examples 1 and 3 respectively were repeated except that a net formed from polymer blend of Ethylene-vinyl acetate copolymer and high impact polystyrene was employed as the intermediate layer between the foam absorbent and the adhesive, instead of the Estane polyurethane net.

### Example 5

An oblate 'window' dressing was produced measuring 67mm x 25mm overall. The thickened central region measured 60mm x 19mm.

The dressing was prepared as described in Example 1 except that the adhesive was an acrylate adhesive as described in European Patent Publication No. 99675.

Prior to coating the adhesive on the transfer sheet, red endorsing ink was mixed into adhesive mass to colour it pink. The adhesive was spread onto the transfer sheet using a 200µ spreading block. Oblate windows measuring 25mm x 12.5mm were cut from the adhesive sheet. The adhesive was then transfer coated onto the foam surface of the absorbent layer, ensuring that the window registered with the central thickened region. The non-adhesive area of the dressing was clearly visible.

## Claims

1. A conformable wound dressing comprising an absorbent layer with a planar wound facing surface comprising a polymeric foam, characterised in that the dressing comprises a wound facing discontinuous adhesive layer over said surface of the absorbent layer and a layer of a liquid impervious moisture vapour permeable material over the opposed surface of the absorbent layer wherein the thickness of the absorbent layer at two opposed margins is substantially less than the thickness of the absorbent layer between said margins.

2. A dressing as claimed in claim 1 which further comprises a discontinuous layer intermediate the absorbent layer and the adhesive.

3. A dressing according to claim 1 or claim 2 wherein the thickness of the absorbent layer at said margins is less than half the thickness of the absorbent layer between the margins.

4. A dressing according to any one of the preceding claims wherein the thickness of the absorbent material between the margins is up to 2.5mm.

5. A dressing as claimed in claim 2 wherein the discontinuous layer is a net.

6. A dressing as claimed in any one of the preceding claims wherein a portion of the absorbent layer between opposed margins is free of adhesive.

7. A dressing as claimed in any one of claims 1 to 5 wherein all the layers are coextensive.

8. A dressing as claimed in any one of the preceding claims wherein the absorbent material is a polyurethane foam.

9. A dressing as claimed in any one of the preceding claims wherein the liquid impervious moisture vapour permeable material is a polyurethane film.

10. A dressing as claimed in claims 2 or 5 wherein the intermediate discontinuous layer is a polyurethane net.

11. A dressing as claimed in any one of the preceding claims in which the margins of the dressing comprise up to 90% of the area of the dressing.

12. A dressing as claimed in any one of the preceding claims wherein the dressing is moisture vapour permeable.

13. A dressing as claimed in claim 12 wherein the moisture vapour permeability of the dressing is from 300 to 5000 grams per square meter per 24 hrs at 37°C and at 100% to 10% relative humidity difference.

14. A dressing as claimed in any one of the preceding claims wherein the absorbent layer contains a medicament.

15. A dressing as claimed in claims 2, 5 or 10 wherein the intermediate discontinuous layer contains a medicament.

16. A process of making a wound dressing of any one of claims 1 to 15 which comprises bringing together a layer of liquid impervious moisture vapour permeable layer, an absorbent layer comprising polymeric foam and a wound facing layer with adhesive on its wound facing surface.

## Patentansprüche

1. Nachgiebiger Wundverband, umfassend eine absorbierende Schicht mit einer ebenen, der Wunde gegenüberliegenden Oberfläche, die einen polymeren Schaum umfaßt, dadurch gekennzeichnet, daß der Verband über der Oberfläche der absorbierenden Schicht eine der Wunde gegenüberliegende, unterbrochene Klebeschicht und über der entgegengesetzten Oberfläche der absorbierenden Schicht eine Schicht aus einem flüssigkeitsundurchlässigen, feuchtigkeitsdampfdurchlässigen Material umfaßt, wobei die Dikke der absorbierenden Schicht an den beiden entgegengesetzten Rändern wesentlich geringer als die Dicke der absorbierenden Schicht zwischen den Rändern ist.

2. Verband wie in Anspruch 1 beansprucht, der weiter eine unterbrochene Schicht zwischen der absorbierenden Schicht und dem Klebstoff umfaßt.

3. Verband gemäß Anspruch 1 oder Anspruch 2, wobei die Dicke der absorbierenden Schicht an den Rändern weniger als die Hälfte der Dicke der absorbierenden Schicht zwischen den Rändern beträgt.

4. Verband gemäß einem der vorangehenden Ansprüche, wobei die Dicke des absorbierenden Materials zwischen den Rändern bis zu 2,5 mm beträgt.

5. Verband wie in Anspruch 2 beansprucht, wobei die unterbrochene Schicht ein Netz ist.

6. Verband wie in einem der vorangehenden Ansprüche beansprucht, wobei ein Teil der absorbierenden Schicht zwischen entgegengesetzten Rändern frei von Klebstoff ist.

7. Verband wie in einem der Ansprüche 1 bis 5 beansprucht, wobei sich alle Schichten in gleicher Weise ausdehnen.

8. Verband wie in einem der vorangehenden Ansprüche beansprucht, wobei das absorbierende Material ein Polyurethanschaum ist.

9. Verband wie in einem der vorangehenden Ansprüche beansprucht, wobei das flüssigkeitsundurchlässige, feuchtigkeitsdampfdurchlässige Material ein Polyurethanfilm ist.

10. Verband wie in Anspruch 2 oder 5 beansprucht, wobei die unterbrochene Zwischenschicht ein Polyurethannetz ist.

11. Verband wie in einem der vorangehenden Ansprüche beansprucht, wobei die Ränder des Verbands bis zu 90% der Fläche des Verbands umfassen.

12. Verband wie in einem der vorangehenden Ansprüche beansprucht, wobei der Verband feuchtigkeitsdampfdurchlässig ist.

13. Verband wie in Anspruch 12 beansprucht, wobei die Feuchtigkeitsdampfdurchlässigkeit des Verbands 300 bis 5000 g je Quadratmeter je 24 h bei 37°C und bei 100% bis 10% relativer Feuchtigkeitsdifferenz beträgt.

14. Verband wie in einem der vorangehenden Ansprüche beansprucht, wobei die absorbierende Schicht ein Arzneimittel enthält.

15. Verband wie in Anspruch 2, 5 oder 10 beansprucht, wobei die unterbrochene Zwischenschicht ein Arzneimittel enthält.

16. Verfahren zum Herstellen eines Wundverbands eines der Ansprüche 1 bis 15, welches das Zusammenbringen einer Schicht aus einer flüssigkeitsundurchlässigen feuchtigkeitsdampfdurchlässigen Schicht, einer einen Polymerschaum umfassenden, absorbierenden Schicht und einer der Wunde gegenüberliegenden Schicht mit einem Klebstoff auf ihrer der Wunde gegenüberliegenden Oberfläche umfaßt.

## Revendications

1. Pansement pour plaie épousant les formes comprenant une couche absorbante avec une surface plane faisant face à la plaie comprenant une mousse polymère, caractérisé en ce que le pansement comprend une couche adhésive discontinue faisant face à la plaie sur cette surface de la couche absorbante et une couche d'un matériau perméable à la vapeur d'eau imperméable aux liquides sur la surface opposée de la couche absorbante dans lequel l'épaisseur de la couche absorbante au niveau de deux marges opposées est notablement inférieure à l'épaisseur de la couche absorbante entre ces marges.

2. Pansement suivant la revendication 1, qui comprend de plus une couche discontinue intermédiaire entre la couche absorbante et l'adhésif.

3. Pansement suivant la revendication 1 ou la revendication 2, dans lequel l'épaisseur de la couche absorbante au niveau de ses marges est inférieure à la moitié de l'épaisseur de la couche absorbante entre les marges.

4. Pansement suivant l'une quelconque des revendications précédentes, dans lequel l'épaisseur du matériau absorbant entre les marges va jusqu'à 2,5 mm.

5. Pansement suivant la revendication 2, dans lequel la couche discontinue est un filet.

6. Pansement suivant l'une quelconque des revendications précédentes, dans lequel une partie de la couche absorbante entre des marges opposées est dépourvue d'adhésif.

7. Pansement suivant l'une quelconque des revendications 1 à 5, dans lequel toutes les couches ont la même étendue.

8. Pansement suivant l'une quelconque des revendications précédentes, dans lequel le matériau absorbant est une mousse de polyuréthane.

9. Pansement suivant l'une quelconque des revendications précédentes, dont le matériau perméable à la vapeur d'eau et imperméable aux liquides est un film de polyuréthane.

10. Pansement suivant les revendications 2 ou 5, dans lequel la couche discontinue intermédiaire est un filet de polyuréthane.

11. Pansement suivant l'une quelconque des revendications précédentes, dans lequel les marges du pansement représentent jusqu'à 90 % de la surface du pansement.

12. Pansement suivant l'une quelconque des revendications précédentes, dans lequel le pansement est perméable à la vapeur d'eau.

13. Pansement suivant la revendication 12, dans lequel la perméabilité à la vapeur d'eau du pansement est comprise entre 300 et 5.000 g/m²/24 heures, à 37°C et à une différence d'humidité relative de 100 % à 10 %.

14. Pansement suivant l'une quelconque des revendications précédentes, dans lequel la couche absorbante contient un médicament.

15. Pansement suivant les revendications 2, 5 ou 10, dans lequel la couche discontinue intermédiaire contient un médicament.

16. Procédé de fabrication d'un pansement pour plaie suivant l'une quelconque des revendications 1 à 15, qui comprend la mise en contact d'une couche de matériau perméable à la vapeur d'eau et imperméable aux liquides, une couche absorbante comprenant une mousse polymère et d'une couche faisant face à la plaie avec un adhésif sur sa surface faisant face à la plaie.
